# EUROPÄISCHE PATENTSCHRIFT

(11) **EP 0 448 954 B1**
(45) Veröffentlichungstag und Bekanntmachung des Hinweises auf die Patenterteilung: **29.06.1994**
(21) Anmeldenummer: 91102348.9
(22) Anmeldetag: 19.02.1991
(51) Int. Cl.: A61M 16/01

(54) **Anordnung zum Füllen und Abzapfen von Anästhesiemittel**
Device for filling and dispensing of anaesthetic
Dispositif de remplissage et distribution d'anesthésique

(30) Priorität: 30.03.1990 SE 9001173
(43) Veröffentlichungstag der Anmeldung: 02.10.1991
(73) Patentinhaber: Siemens-Elema AB, 171 95 Solna 1 (SE); SIEMENS AKTIENGESELLSCHAFT, 80333 München (DE)
(72) Erfinder: Westberg, Hans, S-135 35 Tyresö (SE); Thorsén, Bill, S-197 00 Bro (SE)

(56) Entgegenhaltungen:
- EP-A- 0 295 671
- GB-A- 1 394 216
- US-A- 2 664 084

## Beschreibung

Die Erfindung bezieht sich auf ein Verbindungsteil zum Füllen eines Narkosemittelvergasers mit Anästhesiemittel von einem Behälter bzw. Abzapfen von Anästhesiemittel aus dem Narkosemittelvergaser in den Behälter, das einen Kanal für Anästhesiemittel und einen Kanal für Luft aufweist.

Ein Verbindungsteil dieser Art ist durch die GB-PS 1 394 216 bekannt. Das Verbindungsteil besteht aus einem Kunststoffschlauch, in dem die erwähnten Kanäle verlaufen. Das eine Ende des Kunststoffschlauches ist mit einem Flaschenverschluß versehen, an den eine Flasche mit Anästhesiemittel angeschlossen wird. Das andere Ende des Schlauches weist ein Teil für den Anschluß an den Narkosemittelvergaser auf. Da diese Anordnung kein Ventil zur Regelung des Anesthesiemittelflußes beim Füllen bzw` Abzapfen besitzt, darf der Narkosemittelvergaser nicht in dem Operationsraum gefüllt bzw. entleert werden, da das Risiko eines Entweichens des Anästhesiemittels besteht. Dies erfolgt daher unter einem Abzug außerhalb des Operationsraumes. Außerdem muß der Operateur aufgrund des Aufbaus der Anordnung die Flasche beim Füllen bzw Abzapfen in der richtigen Lage halten.

Der Erfindung liegt die Aufgabe zugrunde, ein Verbindungsteil der eingangs genannten Art zu schaffen, bei der ein Füllen bzw Entleeren eines Narkosemittelvergasers ohne das Risiko eines Entweichens von Anästhesiemittel erfolgen kann. Außerdem soll das Verbindungsteil so aufgebaut sein, daß das Füllen bzw. Abzapfen des Narkosemittelvergasers für den Operateur erleichtert wird.

Diese Aufgabe ist erfindungsgemäß dadurch gelöst, daß das Verbindungsteil aus einem ersten und einem zweiten Gelenkarm besteht, die beide über ein Gelenk gegeneinander schwenkbar verbunden sind, wobei beide Gelenkarme mit jeweils zwei Kanälen versehen sind und wobei die Kanäle des ersten Gelenkarmes mit den Kanälen des zweiten Gelenkarmes verbindbar sind, wenn die Gelenkarme in bestimmte Winkellagen zueinander gebracht werden. Durch den Aufbau des Verbindungsteils bildet dieses gleichzeitig ein Ventil, das die Kanalverbindungen abhängig von den Winkellagen der Gelenkarme zueinander öffnet bzw. schließt. Das wird beim Füllen und Entleeren eines Narkosemittelvergasers benützt. Hierdurch kann ein Entweichen von Anästhesiemittel vermieden werden.

In einer vorteilhaften Ausbildung der Erfindung wird vorgeschlagen, daß das Gelenk der Gelenkarme aus deren Enden gebildet ist, die Überlappungsteile aufweisen, die mit Anlageflächen versehen sind, über welche die Überlappungsteile gegeneinander schwenkbar befestigt sind, und daß das eine Ende jedes Kanales der Gelenkarme in der jeweiligen Anlagefläche mündet, wobei die genannten Kanalenden des ersten Gelenkarmes mit denen des zweiten Gelenkarmes durch Nuten verbindbar sind, die im Überlappungsteil des einen Gelenkarmes angeordnet sind. Hierdurch können die Gelenkarme des Verbindungsteils in bestimmte feste Positionen, wie z.B. in eine Füll- bzw. Abzapfposition oder in eine geschlossene Position gebracht werden. Dies bedeutet, daß der Operateur in diesen Positionen die Flasche nicht halten muß.

Weitere Vorteile und Einzelheiten der Erfindung ergeben sich aus den Unteransprüchen.

Die Erfindung ist nachfolgend anhand eines in den Zeichnungen dargestellten Ausführungsbeispieles näher erläutert. Es zeigen:
- FIG. 1: eine Seitenansicht einer Anordnung nach der Erfindung teils im Schnitt
- FIG. 2: eine Frontansicht der Anordnung nach FIG. 1 und
- FIG. 3 bis 5: die Anordnung nach den FIG. 1 und 2 in verschiedenen Positionen in Verbindung mit dem Anschluß an einen Narkosemittelvergaser.

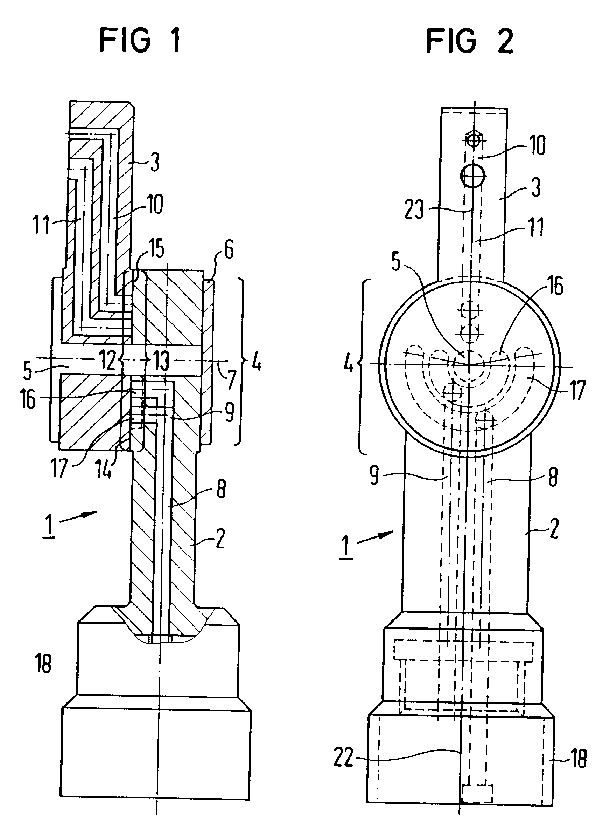

In der FIG. 1 ist eine Anordnung zum Füllen eines in den FIG. 2 bis 5 gezeigten Narkosemittelvergasers mit Anästhesiemittel von einer ebenso in den FIG. 2 bis 5 gezeigten Flasche bzw. zum Abzapfen von Anästhesiemitteln aus dem Narkosemittelvergaser in die Flasche dargestellt. Die Anordnung besteht aus einem Verbindungsteil 1 mit einem ersten Gelenkarm 2 und mit einem zweiten Gelenkarm 3, die in einem Gelenk 4 mit Hilfe einer Achse 5 mit einem Flansch und einer Mutter 6 miteinander schwenkbar verbunden sind. Die Achse 5 ist entlang der Mittellinie 7 des Gelenkes 4 angebracht. Das Gelenk 4 besteht aus den Enden der Gelenkarmen 2,3, die Überlappungsteile 12,13 bilden, die mit Anlageflächen 14,15 versehen sind. Die Gelenkarme 2 und 3 sind mit jeweils zwei Kanälen 8,9 bzw 10,11 versehen. Die Kanäle 8 und 10 dienen als Luftkanäle und die Kanäle 9 und 11 dienen als Anästhesiemittelkanäle. Das eine Ende jedes Kanals 10,11 des Gelenkarmes 3 mündet in dessen Anlagefläche 14. Die anderen Enden münden im Bereich des freien Endes des Gelenkarmes 3. Das eine Ende jedes Kanals 8,9 des Gelenkarmes 2 mündet in einer Nut 16,17, die im Überlappungsteil 12 des Gelenkarmes 2 angeordnet sind. Die anderen Enden der Kanäle 8,9 münden in einem Flaschenverschluß 18, der mit dem freien Ende des Gelenkarmes 2 verbunden ist.

In der FIG. 2 wird gezeigt, daß die Nuten 16,17 kreisförmig gebogen und konzentrisch um die Achse 5 des Gelenks 4 angeordnet sind. In dieser FIG. wird auch dargestellt, daß die Nuten 16,17 so angebracht sind, daß die Kanäle 8,10 und 9,11 der Gelenkarme 2,3 nicht miteinander in Verbindung stehen, wenn die Gelenkarme 2,3 in eine Lage gebracht sind, in der deren Mittelachsen 22,23 achsfluchtend ausgerichtet sind.

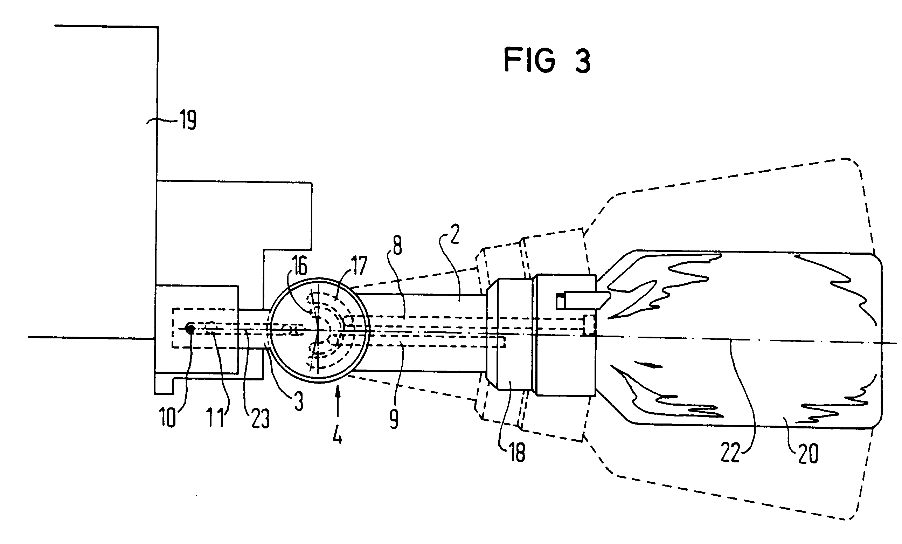

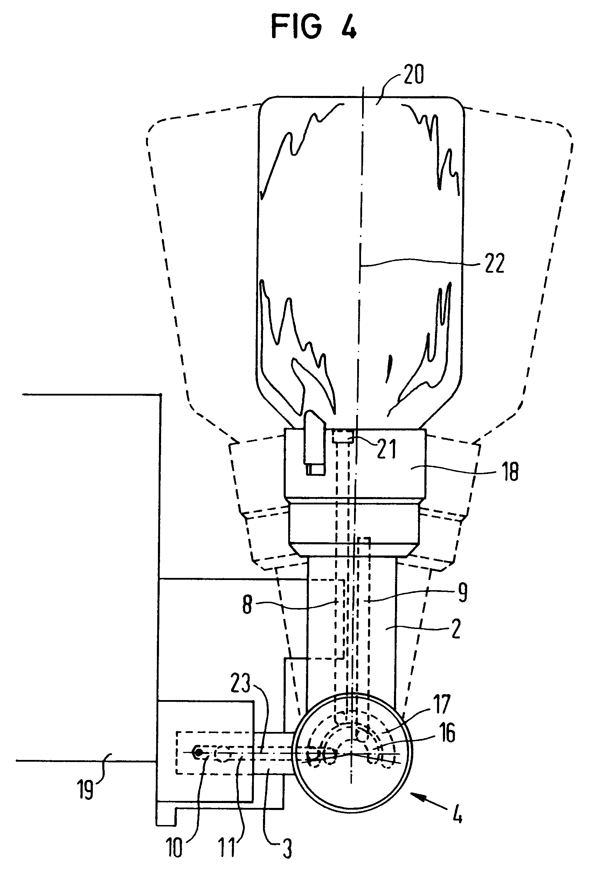

Beim Füllen eines Narkosemittelvergasers (FIG. 3) wird der Flaschenverschluß 18 des Verbindungsteils 1 an einer Flasche 20, die mit Anästhesiemittel gefüllt ist, angeschlossen. Danach wird das freie Ende des Gelenkarmes 3 in eine Öffnung des Narkosemittelvergasers 19 hineingeführt, so daß die Enden der Kanäle 10 und 11 in der Öffnung münden. In der FIG. 3 wird gezeigt, daß beim Anschließen des Verbindungsteils 1 bzw. der Flasche 20 an dem Narkosemittelvergaser 19 das Verbindungsteil 1 in eine Lage gebracht ist, in der die Flüssigkeitsverbindung zwischen der Flasche 20 und dem Narkosemittelvergaser 19 unterbrochen ist. Auf diese Weise kann in der Anschlußphase kein Anästhesiemittel entweichen. Auch wenn der Gelenkarm 2 bzw. die Flasche 20 etwas nach oben oder etwas nach unten im Verhältnis zur Horizontalebene geschwenkt wird, wie es durch die gestrichelten Flaschenkonturen angedeutet ist, ist die Flüssigkeitsverbindung gesperrt. Danach wird der Gelenkarm 2 bzw. die Flasche 20 um das Gelenk 4 in eine vertikale Lage gedreht, so daß die Öffnung der Flasche nach unten zeigt, wie es in der Fig 4 dargestellt ist. In dieser beschriebenen Lage befinden sich die Nuten 16,17 des Gelenkarmes 2 in einer derartigen Position, daß die Mündungen der Kanäle 10,11 des Gelenkarms 3 genau vor die Nuten 16,17 zu liegen kommen. Auf diese Weise ist eine Verbindung zwischen den Kanälen 8,9 des Gelenkarmes 2 und den Kanälen 10,11 des Gelenkarms 3 hergestellt worden. Nun strömt Anästhesiemittel von der Flasche 20 durch den Kanal 9 über die Nut 16 und durch den Kanal 11 zum Narkosemittelvergaser 19. Die Luftkanäle 8 und 10 sowie die Nut 17 verhindern, daß ein Unterdruck in der Flasche 20 entsteht. An dem freien Ende des Luftkanals 8 ist ein Rückschlagventil angeordnet, das verhindert, daß Anästhesiemittel in den Luftkanal eindringt. Die Flasche 20 kann auch in ihrer vertikalen Lage etwas um das Gelenk 4 geschwenkt werden, ohne daß die Verbindung zwischen den Kanälen der Gelenkarme 2 und 3 unterbrochen wird. Die Schwenkung wird durch die gestrichelten Konturen dargestellt.

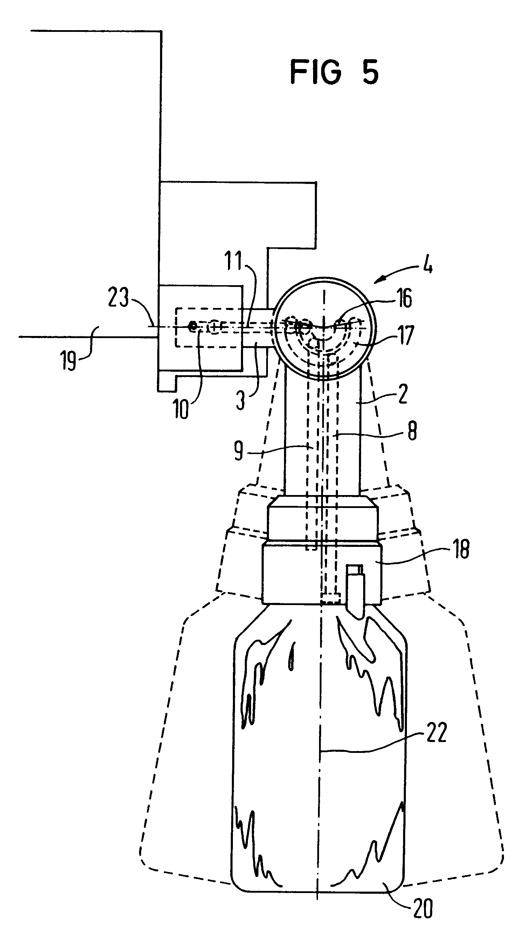

Beim Abzapfen von Anästhesiemittel vom Narkosemittelvergaser 19, dargestellt in der Fig 5, wird der Gelenkarm 2 bzw die Flasche 20 um das Gelenk 4 in eine vertikale Lage geschwenkt, bei der die Öffnung der Flasche nach oben zeigt. Durch diese Lage hat der Gelenkarm eine solche Position eingenommen, daß die Mündungen der Kanäle 10,11 des Gelekarmes 3 genau vor den Nuten 16,17 liegen, so daß eine Verbindung zwischen den Kanälen 8,9 des Gelenkarmes 2 und den Kanälen 10,11 des Gelenkarmes 3 hergestellt ist. Hierdurch kann das Anästhesiemittel vom Narkosemittelvergaser 19 durch den Kanal 11 des Gelenkarmes 3 über den Nut 16 und durch den Kanal 9 des Gelenkarmes 3 zur Flasche 20 strömen. Auch in dieser Lage kann die Flasche 20 etwas um das Gelenk 4 geschwenkt werden, ohne daß die Verbindung zwischen den Kanälen 11 und 19 unterbrochen wird.

Dadurch, daß die Überlappungsteile 12,13 der Gelenkarme 2,3 schwenkbar befestigt gegeneinander anliegen, können die in Verbindung mit den Fig. 3 bis 5 beschriebenen Positionen des Gelenkarmes 2 bzw. der Flasche 20 derart fixiert werden, daß der Operateur beim Füllen bzw. beim Abzapfen die Flasche 20 nicht zu halten braucht.

## Patentansprüche

1. Verbindungsteil (1) zum Füllen eines Narkosemittelvergasers mit Anästhesiemittel von einem Behälter bzw Abzapfen von Anästhesiemittel aus dem Narkosemittelvergaser in den Behälter, das einen Kanal für Anästhesiemittel und einen Kanal für Luft aufweist, **dadurch** **gekennzeichnet,** daß das Verbindungsteil (1) aus einem ersten und einem zweiten Gelenkarm (2,3) besteht, die beide über ein Gelenk (4) gegeneinander
schwenkbar verbunden sind, wobei beide Gelenkarme (2,3) mit jeweils zwei Kanälen (8 bis 11) versehen sind und wobei die Kanäle (8,9) des ersten Gelenkarmes (2) mit den Kanälen (10,11) des zweiten Gelenkarmes (3) verbindbar sind, wenn die Gelenkarme (2,3) in bestimmte Winkellagen zueinander gebracht werden.

2. Verbindungsteil nach Anspruch 1, **dadurch gekennzeichnet**, daß das Gelenk (4) der Gelenkarme (2,3) aus deren Enden gebildet ist, die Überlappungsteile (12,13) aufweisen, die mit Anlageflächen (14,15) versehen sind, über welche die Überlappungsteile (12,13) gegeneinander anliegend schwenkbar befestigt sind, und daß das eine Ende jedes Kanales (8 bis 11) der Gelenkarme (2,3) in der jeweiligen Anlagefläche mündet, wobei die genannten Kanalenden des ersten Gelenkarmes (2) mit den genannten Kanalenden des zweiten Gelenkarmes (3) durch Nuten (16,17) verbindbar sind, die im Überlappungsteil (12,13) des einen Gelenkarmes (2,3) angeordnet sind.

3. Verbindungsteil nach Anspruch, **dadurch gekennzeichnet**, daß die Nuten (16,17) kreisförmig gebogen und konzentrisch um die Achse (5) des Gelenks (4) angordnet sind.

4. Verbindungsteil nach einem der Ansprüche 2 bis 3, **dadurch** **gekennzeichnet,** daß die Nuten (16,17) derart angeordnet sind, daß die Kanäle (8 bis 11) der beiden Gelenkarme (2,3) nicht miteinander in Verbindung stehen, wenn die Gelenkarme (2,3) in eine Lage gebracht sind, bei der deren Mittelachsen (22,23) achsfluchtend liegen.

## Claims

1. Connection part (1) for filling an anaesthetic vaporizer with an anaesthetic from a container and dispensing anaesthetic from the anaesthetic vaporizer into the container, which connection part has a channel for an anaesthetic and a channel for air, characterized in that the connection part (1) consists of a first articulated arm (2) and a second articulated arm (3) which are both connected in a pivotable manner relative to one another via a hinge (4), both articulated arms (2, 3) being provided with in each case two channels (8 to 11), and it being possible for the channels (8, 9) of the first articulated arm (2) to be connected to the channels (10, 11) of the second articulated arm (3) when the articulated arms (2, 3) are brought into defined angular positions with respect to one another.

2. Connection part according to Claim 1, characterized in that the hinge (4) of the articulated arms (2, 3) is formed by the ends of the articulated arms (2, 3), which ends have overlapping parts (12, 13) provided with contact surfaces (14, 15) by means of which the overlapping parts (12, 13) are secured bearing against one another in a pivotable manner, and in that the one end of each channel (8 to 11) of the articulated arms (2, 3) opens out in the respective contact surface, it being possible for the said channel ends of the first articulated arm (2) to be connected to the said channel ends of the second articulated arm (3) through grooves (16, 17), which are arranged in the overlapping part (12, 13) of the one articulated arm (2, 3).

3. Connection part according to Claim 2, characterized in that the grooves (16, 17) are curved in a circular configuration and are arranged concentrically about the axis (5) of the hinge (4).

4. Connection part according to one of Claims 2 to 3, characterized in that the grooves (16, 17) are arranged in such a way that the channels (8 to 11) of the two articulated arms (2, 3) do not communicate with one another when the articulated arms (2, 3) are brought into a position in which their central axes (22, 23) lie axially aligned.

## Revendications

1. Pièce de liaison destinée à remplir un dispositif de mise en phase gazeuse d'un produit narcotique d'un anesthésique provenant d'un récipient ou a le soutirer du dispositif dans le récipient, qui comporte un canal pour l'anesthésique et un canal pour l'air, caractérisée en ce qu'elle est constituée d'un premier bras articulé et d'un second bras articulé (2, 3) qui sont tous les deux reliés ensemble de façon pivotante par l'intermédiaire d'une articulation (4), les deux bras articulés (2, 3) étant munis chacun de deux canaux (8 à 10) et les canaux (8, 9) du premier bras articulé (2) pouvant être mis en communication avec les canaux (10, 11) du second bras articulé (3) lorsque les bras articulés (2, 3) sont placés l'un par rapport à l'autre dans des positions angulaires déterminées.

2. Pièce de liaison selon la revendication 1, caractérisée en ce que l'articulation (4) des bras articulés (2, 3) est formée à leurs extrémités, qui présentent des parties (12, 13) de chevauchement qui sont munies de surfaces d'appui (14, 15) par lesquelles les parties (12, 13) de chevauchement sont fixées de façon pivotante, en appui l'une sur l'autre, et en ce que l'une des extrémités de chaque canal (8 à 11) des bras articulés (2, 3) débouche dans la surface d'appui (8 à 11) correspondante, lesdites extrémités de canal du premier bras articulé (2) pouvant être mises en communication avec lesdites extrémités de canal du second bras articulé (3) par des gorges (16, 17) qui sont disposées dans la partie (12, 13) de chevauchement de l'un des bras articulés (2, 3).

3. Pièce de liaison selon la revendication 2, caractérisée en ce que les gorges (16, 17) présentent une courbure circulaire et sont concentriques par rapport à l'axe (5) de l'articulation (4).

4. Pièce de liaison selon la revendication 2 ou 3, caractérisée en ce que les gorges (16, 17) sont disposées de telle manière que les canaux (8 à 11) des deux bras articulés (2, 3) ne communiquent pas entre eux lorsque les bras articulés (2, 3) sont amenés dans une position dans laquelle leurs axes médians (22, 23) sont en alignement.
